(19) Europäisches Patentamt
European Patent Office
Office européen des brevets

(11) **EP 4 678 200 A1**

(12) **EUROPEAN PATENT APPLICATION**
published in accordance with Art. 153(4) EPC

(43) Date of publication:
**14.01.2026 Bulletin 2026/03**

(21) Application number: **24811181.7**

(22) Date of filing: **23.05.2024**

(51) International Patent Classification (IPC):
**A61M 1/16** (2006.01)　　**A61M 1/14** (2006.01)

(52) Cooperative Patent Classification (CPC):
**A61M 1/14; A61M 1/16**

(86) International application number:
**PCT/JP2024/019067**

(87) International publication number:
**WO 2024/242176 (28.11.2024 Gazette 2024/48)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: **25.05.2023 JP 2023086341**

(71) Applicant: **Physiologas Technologies, Inc.
Sagamihara-shi, Kanagawa 252-0373 (JP)**

(72) Inventors:
• **MIYAWAKI, Kazuyoshi
Sagamihara-shi, Kanagawa 252-0373 (JP)**
• **KOKUBO, Kenichi
Sagamihara-shi, Kanagawa 252-0373 (JP)**
• **AOKI, Hiroyuki
Sagamihara-shi, Kanagawa 252-0373 (JP)**

(74) Representative: **Schön, Christoph
Dr. Schön, Neymeyr & Partner mbB
Bavariaring 26
80336 München (DE)**

(54) **HEMODIALYSIS TREATMENT ASSISTANCE SYSTEM**

(57)　A system capable of improving an evaluation accuracy of an outcome of hemodialysis treatment is provided. A peak value of a concentration of a designated substance is obtained according to a kinetic model based on treatment information including a clearance of a dialyzer used for hemodialysis treatment of a patient in a designated period, a time of the hemodialysis treatment for each session and the number of times of the hemodialysis treatment, and a body fluid volume of the patient. An outcome of the hemodialysis treatment for the patient in the designated period is evaluated based on an HDP or an sf-Kt/V corresponding to the peak value.

FIG.1

EP 4 678 200 A1

**Description**

Technical Field

The present invention relates to a technique for assisting blood purification treatment by a medical professional.

Background Art

In the related art, a technique for generating renal failure blood medical examination prescriptions for patients has been proposed (see, for example, Patent Literature 1). Specifically, parameters of a kinetic model specific to a patient are first identified based on a concentration of a solute to be removed (such as urea, $\beta$2-M and/or phosphate) in blood samples taken from the patient a plurality of times. A target concentration of the solute in the patient's blood is then input into this kinetic model. As a result, a medical examination prescription, including a duration of the medical examination, a frequency of the medical examination, dialysis fluid flow rate and/or blood flow rate to achieve the target concentration of the solute, is proposed. In addition, a patient's lifestyle is taken into account in the medical examination prescription to determine a final method of the medical examination.

On the other hand, there are an indicator called HDP that determines excess and deficiency of dialysis from the number of times of dialysis per week and the dialysis time, and an indicator called sf-Kt/V that further considers a body weight or a removal efficiency (clearance) of a dialyzer, and these are able to be calculated relatively easily and used.

Citation List

Patent Literature

Patent Literature 1: Japanese Patent No. 6018567

Non-Patent Literatures

Non-Patent Literature 1: Scribner BH, Oreopolus DG: The Hemodialysis product (HDP): A better index of dialysis adequacy than Kt/V. Dial Transplant 31: 13-15, 2002
Non-Patent Literature 2: Squared frequency-Kt/V: a new index of hemodialysis adequacy-correlation with solute concentrations by computer simulation, Murakami et al. Renal Replacement Therapy (2019) 5:8 (https://doi.org/10.1186/s41100-019-0198-7)

Summary of Invention

Technical Problem

In a case where the kinetic model is used, blood data of the collected blood or data measured using a sensor related to the treatment of the patient is used, but the accuracy or certainty of a calculation processing result may be reduced due to a measurement error or the like. Since the HDP does not consider a body weight or a removal performance in dialysis, the value may deviate significantly depending on the treatment method or the patient. Although these factors are considered in the sf-Kt/V, neither the HDP nor the sf-Kt/V is able to be applied in a case where the treatment time varies for each treatment or the dialysis day varies for each week.

Therefore, an object of the present invention is to provide a system that is able to improve the evaluation accuracy of the outcomes of hemodialysis treatment.

Solution to Problem

A hemodialysis treatment assistance system of the present invention is configured to obtain a peak value of a concentration of a designated substance according to a kinetic model based on treatment information including a clearance of a dialyzer used for hemodialysis treatment of a patient in a designated period, a time of the hemodialysis treatment for each session and the number of times of the hemodialysis treatment in the designated period, and a body fluid volume of the patient, and evaluate an outcome of the hemodialysis treatment for the patient in the designated period based on an HDP or an sf-Kt/V corresponding to the peak value.

In the hemodialysis treatment assistance system having the above configuration, it is preferable that the outcome of the hemodialysis treatment for the patient is evaluated in at least one of a first designated period which is a past designated period of the patient and a second designated period which is a future designated period of the patient, based on at least one of first treatment information as the treatment information representing a treatment history in the first designated period, and second treatment information as the treatment information representing a treatment schedule in the second designated period.

In the hemodialysis treatment assistance system having the above configuration, it is preferable that the HDP or the sf-Kt/V is corrected in a manner in which the HDP or the sf-Kt/V is decreased as a variation of at least one of the time of the hemodialysis treatment for each session, a time interval during which the hemodialysis treatment is not performed, and the number of times of the hemodialysis treatment in the designated period is increased (non-uniform treatment).

In the hemodialysis treatment assistance system having the above configuration, it is preferable that an evaluation result of the outcome of the hemodialysis treatment for the patient in the designated period is output to an output interface.

Brief Description of Drawings

FIG. 1 is a diagram illustrating a configuration of a hemodialysis treatment assistance system according to an embodiment of the present invention.

FIG. 2 is a functional explanatory diagram of the hemodialysis treatment assistance system according to an embodiment of the present invention.

Description of Embodiments

(Configuration)

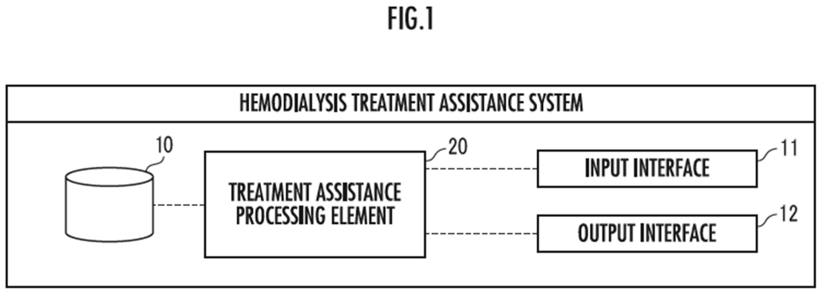

A hemodialysis treatment assistance system according to an embodiment of the present invention illustrated in FIG. 1 includes a database 10, an input interface 11, an output interface 12, and a treatment assistance processing element 20.

The database 10 is configured to store and hold a measurement result, a model, and the like representing a state of the patient which will be described later. The database 10 may include a database server separate from the hemodialysis treatment assistance system.

The input interface 11 includes a keyboard, a touch panel, an imaging apparatus, and/or a microphone (audio input apparatus), and is configured to receive an input of information related to the patient in response to keyboard operations, operations such as touch, tap, swipe, and pinch, gestures, and/or speech of a user such as a medical professional.

The output interface 12 includes a monitor (image display apparatus, touch panel type display) and/or a speaker, and is configured to output information for assisting the medical examination of the patient by the user such as the medical professional.

The treatment assistance processing element 20 includes an arithmetic processing apparatus (CPU, processor, and/or processor core, or the like), a storage apparatus (memory such as ROM and RAM, HDD, SSD, or the like), an interface circuit, and the like. The treatment assistance processing element 20 is configured to execute various types of arithmetic processing described below by reading out necessary data and program (software) from the storage apparatus (which may constitute the database 10) by the arithmetic processing apparatus and executing the arithmetic processing on the data according to the program.

(Functions)

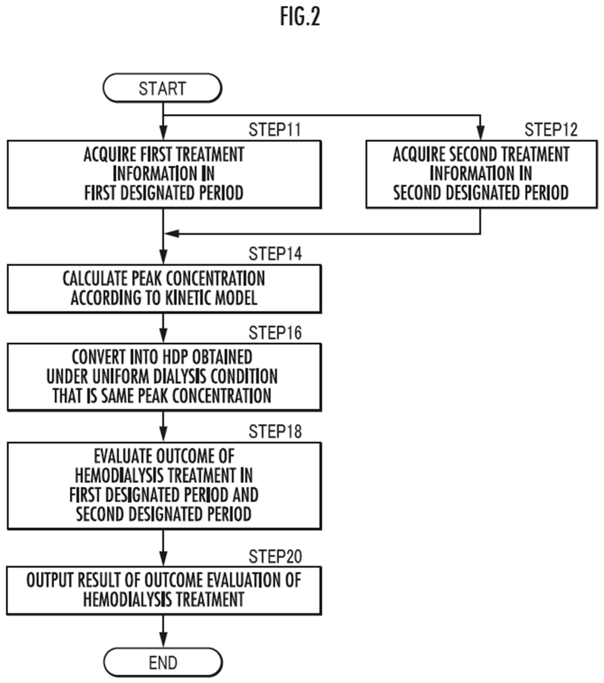

First treatment information representing a treatment history in a first designated period (for example, a plurality of days such as one week until the day or the previous day) which is a designated past period of the patient is acquired through the input interface 11 (FIG. 2/STEP 11). The first treatment information includes a clearance of a dialyzer used for the hemodialysis treatment of the patient, a time of the hemodialysis treatment for each session and the number of times thereof, and a body fluid volume of the patient. The time of the hemodialysis treatment for each session, the number of times thereof, and the like included in the first treatment information may or may not be uniform.

Second treatment information representing a treatment schedule in a second designated period (for example, a plurality of days such as one week from the day or the next day) which is a designated future period of the patient is acquired through the input interface 11 (FIG. 2/STEP 12). The second treatment information includes, similarly to the first treatment information, the clearance of the dialyzer used for the hemodialysis treatment of the patient, the time of the hemodialysis treatment for each session and the number of times thereof, and the body fluid volume of the patient. The time of the hemodialysis treatment for each session, the number of times thereof, and the like included in the second treatment information may or may not be uniform.

Based on each of the first treatment information and the second treatment information, a peak value of a concentration of a designated substance (for example, at least one substance of a uremic substance and/or a uremic toxin substance (K, Ca, Na, phosphate, and a free small-molecular substance (urea, creatinine, uric acid, or the like))) is obtained in each of the first designated period and the second designated period (and a single designated period including the first designated period and the second designated period) according to a kinetic model (FIG. 2/STEP 14). As the kinetic model, for example, a single-pool model, a two-pool model, or a local blood flow model is adopted. For example, the peak value is able to be calculated using the kinetic model (see "Kinetic Model for Urea" and "Kinetic model for β2-microglobulin" in Non-Patent Literature 1) according to the method described in "Methods" in Non-Patent Literature 1.

Subsequently, the peak values of the concentration of the designated substance in each of the first designated period and the second designated period are converted into the hemodialysis product (HDP) or Squared Frequency Kt/V (sf-Kt/V) under the condition that the treatment conditions are uniform (FIG. 2/STEP 16). For example, according to the method described in Non-Patent Literature 1, the peak value is able to be converted into HDP or sf-Kt/V (see "FIG. 4" in Non-Patent Literature 1 or the like).

The HDP is obtained according to Relational Expression (1) using the number of times of dialysis n per week and the dialysis time t (hr) per time.

$$HDP = n^\gamma t \ (1 < \gamma. \quad \text{For example, } \gamma = 2.) \quad (1).$$

Sf-Kt/V is obtained according to Relational Expression (2) using the number of times of dialysis n per week, the dialysis time t (hr) per time, the clearance K (catalog data), and the body weight W (kg).

$$Sf - Kt/V = n^2 \cdot CL \cdot t/(W \times 0.6 \times 1000) \quad (2).$$

For the body fluid volume V (mL), other estimation expressions are able to be used other than calculating from 0.6 times the body weight. In addition, a distribution volume of the corresponding substance may be used.

The clearance CL representing the performance of the dialyzer under the dialysis conditions is obtained according to the following Relational Expression (3), for example, using a blood flow rate $Q_B$, a dialysis fluid flow rate $Q_D$, and a value of a performance coefficient $K_0A$ obtained from the catalog data.

$$CL = Q_B(1 - X)/\{(Q_B/Q_D) - X\},$$

$$X = \exp(K_0A(1/Q_B - 1/Q_D)) \quad (3).$$

Table 1 shows the HDP according to the number of times of dialysis n per week and the dialysis time t (hr) per time, and the determination result of whether the dialysis treatment of the patient is sufficient (the presence of "*" represents that the dialysis treatment is sufficient, and the absence of "*" represents that the dialysis is insufficient).

[Table 1]

| t [hr] | Determination of deficiency of dialysis with reference to HDP | | | | | |
|---|---|---|---|---|---|---|
|  | n=2 | n=3 | n=4 | n=5 | n=6 | n=7 |
| 7 | 28 | 63 | *112 | *175 | *252 | *343 |
| 6 | 24 | 54 | *96 | *150 | *216 | *294 |
| 5 | 20 | 45 | *80 | *125 | *180 | *245 |
| 4 | 16 | 36 | 64 | *100 | *144 | *196 |
| 3 | 12 | 27 | 48 | *75 | *108 | *147 |
| 2 | 8 | 18 | 32 | 50 | *72 | *98 |
| 1 | 4 | 9 | 16 | 25 | 36 | 49 |

In the Sf-Kt/V, a similar table is calculated for each body fluid volume V calculated from the body weight of the patient and clearance CL in the treatment of each treatment condition. The body fluid volume V of the patient may be evaluated according to Watson Expressions (41) and (42) using the age Age, the height H (cm), and the dry weight DW (kg) of the patient, in addition to being evaluated as 60% of the body weight of the patient.

(Case where patient is male)

$$\text{Watson V} = 2.447 - 0.09516 \text{ x Age} + 0.1074 \text{ x H} + 0.3362 \text{ x DW} \quad (41).$$

(Case where patient is female)

$$\text{Watson V} = -0.2097 + 0.1069 \text{ x H} + 0.2466 \text{ x DW} \quad (42).$$

On the other hand, since Relational Expressions (1) to (3) are based on the premise that the dialysis time for each session and the dialysis time per week are always the same, Relational Expressions (1) to (3) are not established under non-uniform treatment conditions. Since these values are values depending on the peak concentration, not the average value (time average, integral average, or the like) of the solute concentrations, Relational Expressions (1) to (3) are not established in the non-uniform treatment conditions even though the average value is used.

In order to solve this problem, a method of determining the excess and deficiency of the treatment in the non-uniform treatment is also examined, the peak concentration is obtained in the pharmacokinetic model using the kinetic model, and the peak concentration is converted into HDP or sf-Kt/V in the reference.

Then, the outcome of the hemodialysis treatment of the patient in each of the first designated period and the second designated period are evaluated (FIG. 2/STEP 18). Specifically, determination processing of whether the HDP of the patient is equal to or greater than a predetermined value (for example, 72) or determination processing of whether the sf-Kt/V of the patient is equal to or greater than a predetermined value (for example, 23) is executed.

The result of the outcome evaluation of the hemodialysis treatment in each of the first designated period and the second designated period (as well as the single designated period including the first designated period and the second designated period) is output through the output interface 12 (FIG. 2/STEP 20).

As a result, the user such as the medical professional is able to perceive the propriety of the hemodialysis treatment in each of the first designated period and the second designated period (as well as the single designated period including the first designated period and the second designated period). Further, it is possible to take measures such as changing the schedule (the treatment time for each session and the number of times of treatment) of the hemodialysis treatment in the second designated period as necessary.

(Other Embodiments of Present Invention)

In the embodiment described above, both the first treatment information and the second treatment information are input to the kinetic model, but in another embodiment, only one of the first treatment information or the second treatment information may be input to the kinetic model.

The HDP may be evaluated according to Relational Expression (11) based on a decrease function $\eta_1 = f_1(\alpha_1)$ ($\partial f_1/\partial \alpha_1 < 0$) in which an index value $\alpha_1$ (for example, variance and/or standard deviation) representing a variation in dialysis time t per time, a time interval from a previous end time of dialysis to a current start time of dialysis, and/or the number of times n of dialysis per week is a main variable.

$$\text{HDP} = \eta_1 n^\gamma t \quad (11).$$

The Sf-Kt/V may be evaluated according to Relational Expression (12) based on a decrease function $\eta_2 = f_2(\alpha_2)$ ($\partial f_2/\partial \alpha_2 < 0$) in which an index value $\alpha_2$ (for example, variance and/or standard deviation) representing a variation in dialysis time t per time, a time interval from a previous end time of dialysis to a current start time of dialysis, and/or the number of times n of dialysis per week is a main variable.

$$\text{Sf-Kt/V} = \eta_2 n^2 \cdot \text{CL} \cdot t/(W \text{ x } 0.006) \quad (12).$$

$\eta_1$ and $\eta_2$ may be created using a relational expression obtained in advance by the kinetic model.

In addition, the HDP and/or the sf-Kt/V may be calculated from the converted value of the peak concentration using the kinetic model. That is, the peak concentration $\mu_1$ when dialysis is carried out at a uniform dialysis time and a uniform dialysis interval, and the HDP or the sf-Kt/V at that time are determined by Reference Expression (13) or (14).

$$HDP = f_3(\mu_1) \quad (13).$$

$$sf\text{-}Kt/V = f_4(\mu_1) \quad (14).$$

On the other hand, the peak concentration under the dialysis conditions of an actual non-uniform dialysis time and a dialysis interval is able to be calculated using the kinetic model (for example, the method described in Non-Patent Literature 2). The method is a method of obtaining the converted value of the HDP or the sf-Kt/V by substituting the actual non-uniform dialysis time and peak concentration $\mu_2$ in the dialysis interval, which are calculated using the kinetic model, into the reference expression represented by Reference Expression (13) or (14).

As the function $f_3$ in Reference Expression (13), for example, a power function of $\mu_1$ may be approximately used as represented by the following Expression (130).

$$HDP = a\,\mu_1^{\,n} \quad (130).$$

Similarly, as the function $f_4$ in Reference Expression (14), for example, a power function of $\mu_1$ may be approximately used as represented by the following Expression (140).

$$sf\text{-}Kt/V = b\,\mu_1^{\,n} \quad (140).$$

In this case, a method of calculating the HDP or the Sf-Kt/V at the non-uniform dialysis time and dialysis interval using the average concentration for week instead of the peak concentrations $\mu_1$ and $\mu_2$ and using a relational expression (Equation corresponding to Relational Expressions (13) and (14)) at the uniform dialysis time and dialysis interval may be adopted.

Description of Reference Numerals

10    database
11    input interface
12    output interface
20    treatment assistance processing element

**Claims**

1. A hemodialysis treatment assistance system configured to obtain a peak value of a concentration of a designated substance according to a kinetic model based on treatment information including a clearance of a dialyzer used for hemodialysis treatment of a patient in a designated period, a time of the hemodialysis treatment for each session and the number of times of the hemodialysis treatment in the designated period, and a body fluid volume of the patient, and evaluate an outcome of the hemodialysis treatment for the patient in the designated period based on an HDP or an sf-Kt/V corresponding to the peak value.

2. The hemodialysis treatment assistance system according to Claim 1, wherein
the outcome of the hemodialysis treatment for the patient is evaluated in at least one of a first designated period which is a past designated period of the patient and a second designated period which is a future designated period of the patient, based on at least one of first treatment information as the treatment information representing a treatment history in the first designated period, and second treatment information as the treatment information representing a treatment schedule in the second designated period.

3. The hemodialysis treatment assistance system according to Claim 1, wherein
the HDP or the sf-Kt/V is corrected in a manner in which the HDP or the sf-Kt/V is decreased as a variation of at least one of the time of the hemodialysis treatment for each session, a time interval during which the hemodialysis treatment is not performed, and the number of times of the hemodialysis treatment in the designated period is increased.

4. The hemodialysis treatment assistance system according to any one of Claims 1 to 3, wherein
an evaluation result of the outcome of the hemodialysis treatment for the patient in the designated period is output to an

output interface.

FIG.1

HEMODIALYSIS TREATMENT ASSISTANCE SYSTEM

10

20

TREATMENT ASSISTANCE
PROCESSING ELEMENT

11

INPUT INTERFACE

12

OUTPUT INTERFACE

FIG.2

```
                        ┌──────────────┐
                        │    START     │
                        └──────┬───────┘
                               ├──────────────────────────────┐
                               │                              │
                    STEP11     ▼                   STEP12      ▼
        ┌──────────────────────────────┐   ┌──────────────────────────────┐
        │     ACQUIRE FIRST TREATMENT   │   │    ACQUIRE SECOND TREATMENT   │
        │        INFORMATION IN         │   │        INFORMATION IN         │
        │     FIRST DESIGNATED PERIOD   │   │    SECOND DESIGNATED PERIOD    │
        └──────────────┬───────────────┘   └──────────────┬───────────────┘
                       │◄─────────────────────────────────┘
                STEP14 ▼
        ┌──────────────────────────────┐
        │   CALCULATE PEAK CONCENTRATION│
        │     ACCORDING TO KINETIC MODEL│
        └──────────────┬───────────────┘
                STEP16 ▼
        ┌──────────────────────────────┐
        │    CONVERT INTO HDP OBTAINED  │
        │  UNDER UNIFORM DIALYSIS       │
        │  CONDITION THAT IS SAME       │
        │       PEAK CONCENTRATION      │
        └──────────────┬───────────────┘
                STEP18 ▼
        ┌──────────────────────────────┐
        │      EVALUATE OUTCOME OF      │
        │    HEMODIALYSIS TREATMENT IN  │
        │  FIRST DESIGNATED PERIOD AND  │
        │   SECOND DESIGNATED PERIOD    │
        └──────────────┬───────────────┘
                STEP20 ▼
        ┌──────────────────────────────┐
        │ OUTPUT RESULT OF OUTCOME      │
        │ EVALUATION OF                 │
        │ HEMODIALYSIS TREATMENT        │
        └──────────────┬───────────────┘
                       ▼
                ┌──────────────┐
                │     END      │
                └──────────────┘
```

## INTERNATIONAL SEARCH REPORT

| International application No. |
|---|
| **PCT/JP2024/019067** |

**A. CLASSIFICATION OF SUBJECT MATTER**

*A61M 1/16*(2006.01)i; *A61M 1/14*(2006.01)i
FI:   A61M1/16 117; A61M1/14 110

According to International Patent Classification (IPC) or to both national classification and IPC

**B. FIELDS SEARCHED**

Minimum documentation searched (classification system followed by classification symbols)

A61M1/16; A61M1/14

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| Y | JP 2016-535644 A (BAXTER INTERNATIONAL INCORPORATED) 17 November 2016 (2016-11-17)<br>        paragraphs [0036]-[0317], fig. 1-31 | 1-2, 4 |
| A | | 3 |
| Y | MURAKAMI, Kaya, KOKUBO, Kenichi, HIROSE, Minoru, KOBAYASHI, Kozue, KOBAYASHI, Hirosuke, Squared frequency-Kt/V: a new index of hemodialysis adequacy - correlation with solute concentrations by computer simulation, Renal Replacement Therapy, 19 February 2019, https://rrtjournal.biomedcentral.com/articles/10.1186/s41100-019-0198-7<br>        pages 2-9 | 1-2, 4 |
| A | | 3 |

☐ Further documents are listed in the continuation of Box C.          ☑ See patent family annex.

| | |
|---|---|
| *    Special categories of cited documents:<br>"A"   document defining the general state of the art which is not considered to be of particular relevance<br>"D"   document cited by the applicant in the international application<br>"E"   earlier application or patent but published on or after the international filing date<br>"L"   document which may throw doubts on priority claim(s) or which is cited to establish the publication date of another citation or other special reason (as specified)<br>"O"   document referring to an oral disclosure, use, exhibition or other means<br>"P"   document published prior to the international filing date but later than the priority date claimed | "T"   later document published after the international filing date or priority date and not in conflict with the application but cited to understand the principle or theory underlying the invention<br>"X"   document of particular relevance; the claimed invention cannot be considered novel or cannot be considered to involve an inventive step when the document is taken alone<br>"Y"   document of particular relevance; the claimed invention cannot be considered to involve an inventive step when the document is combined with one or more other such documents, such combination being obvious to a person skilled in the art<br>"&"   document member of the same patent family |

| Date of the actual completion of the international search | Date of mailing of the international search report |
|---|---|
| **10 June 2024** | **25 June 2024** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (July 2022)

## INTERNATIONAL SEARCH REPORT
### Information on patent family members

| Patent document cited in search report | Publication date (day/month/year) | Patent family member(s) | Publication date (day/month/year) |
|---|---|---|---|
| JP 2016-535644 A | 17 November 2016 | WO 2015/026542 A1 paragraphs [0067]-[00349], fig. 1-31 EP 3229159 B1 | |

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 6018567 B

**Non-patent literature cited in the description**

- **SCRIBNER BH** ; **OREOPOLUS DG**. The Hemodialysis product (HDP): A better index of dialysis adequacy than Kt/V. *Dial Transplant*, 2002, vol. 31, 13-15

- **MURAKAMI et al.** Squared frequency-Kt/V: a new index of hemodialysis adequacy-correlation with solute concentrations by computer simulation. *Renal Replacement Therapy*, 2019, vol. 5, 8, https://doi.org/10.1186/s41100-019-0198-7